# EUROPEAN PATENT SPECIFICATION

(11) **EP 4 126 207 B1**
(45) Date of publication and mention of the grant of the patent: **11.03.2026**
(21) Application number: 20719908.4
(22) Date of filing: 31.03.2020
(51) Int. Cl.: A61N 1/375, A61N 1/05, A61N 1/36

(54) **HEADPIECES AND IMPLANTABLE COCHLEAR STIMULATION SYSTEMS INCLUDING THE SAME**
KOPFSTÜCKE UND IMPLANTIERBARE COCHLEASTIMULATIONSSYSTEME DAMIT
MODULES EXTERNES ET SYSTÈMES DE STIMULATION COCHLÉAIRE IMPLANTABLES COMPRENANT CES DERNIERS

(43) Date of publication of application: 08.02.2023
(73) Proprietor: Advanced Bionics AG, 8712 Staefa (CH)
(72) Inventor: SMITH, James, George Elcoate, Santa Clarita, CA 91350 (US); HEERLEIN, Markus, Michael, Valencia, CA 91354 (US)
(74) Representative: Schwan Schorer & Partner mbB
(86) International application number: PCT/US2020/025985
(87) International publication number: WO 2021/201845

(56) References cited:
- EP-A1- 2 853 287
- WO-A1-2019/083540
- US-B2- 8 515 112

## Description

### BACKGROUND

### 1. Field

The present disclosure relates generally to implantable cochlear stimulation (or "ICS") systems.

### 2. Description of the Related Art

ICS systems are used to help the profoundly deaf perceive a sensation of sound by directly exciting the intact auditory nerve with controlled impulses of electrical current. Ambient sound pressure waves are picked up by an externally worn microphone and converted to electrical signals. The electrical signals, in turn, are processed by a sound processor, converted to a pulse sequence having varying pulse widths, rates, and/or amplitudes, and transmitted to an implanted receiver circuit of the ICS system. The implanted receiver circuit is connected to an implantable electrode array that has been inserted into the cochlea of the inner ear, and electrical stimulation current is applied to varying electrode combinations to create a perception of sound. The electrode array may, alternatively, be directly inserted into the cochlear nerve without residing in the cochlea. A representative ICS system is disclosed in U.S. Patent No. 5,824,022, which is entitled "Cochlear Stimulation System Employing Behind-The-Ear Sound processor With Remote Control". Examples of commercially available ICS sound processors include, but are not limited to, the Harmony^{™} BTE sound processor, the Naida^{™} CI Q Series sound processor and the Neptune^{™} body worn sound processor, which are available from Advanced Bionics.

As alluded to above, some ICS systems include an implantable cochlear stimulator (or "cochlear implant"), a sound processor unit, a battery, and a microphone that is part of, or is in communication with, the sound processor unit. The cochlear implant communicates with the sound processor unit, and some ICS systems include a headpiece that is in communication with both the sound processor unit (e.g., a body worn processor or behind-the-ear processor) and the cochlear implant. The headpiece communicates with the cochlear implant by way of a transmitter (e.g., an antenna) on the headpiece and a receiver (e.g., an antenna) on the implant. The headpiece and the cochlear implant may include respective magnets (or respective pluralities of magnets) that are attracted to one another, thereby retaining the headpiece on the head and maintaining the position of the headpiece transmitter on the head over the implant receiver. The skin and subcutaneous tissue that separates the headpiece magnet and implant magnet is sometimes referred to as the "skin flap." In other instances, all of the external components (e.g., the battery, microphone, sound processor, antenna coil and magnet) are carried within a single headpiece. Examples of such systems are disclosed in U.S. Pat. No. 8,811,643, which is entitled "Integrated Cochlear Implant Headpiece," and U.S. Pat. No. 8,515,112, which is entitled "Modular Speech Processor Headpiece".

US8,515,112 B2 discloses a modular headpiece for a cochlear implant including an implant antenna and an implant magnet. The headpiece includes a core and a modular ring. The core includes a signal processor, a transmitter and a magnet that aligns the transmitter with the implant antenna. The modular ring may include an RF receiver and may be used to connect the core to a power source or other accessories, as well as a programming or fitting device.

One issue associated with cochlear implants is compatibility with magnetic resonance imaging ("MRI") systems. For example, the magnets in many conventional cochlear implants are disk-shaped and have north and south magnetic dipoles that are aligned in the axial direction of the disk. Such magnets produce a magnetic field that is perpendicular to the patient's skin and parallel to the axial direction, and this magnetic field direction is not aligned with, and may be perpendicular to, the direction of the MRI magnetic field (typically 1.5 Tesla or more). The misalignment of the interacting magnetic fields may result in demagnetization of the implant magnet or generate a significant amount of torque on the implant magnet that can dislodge the implant magnet and induce tissue damage.

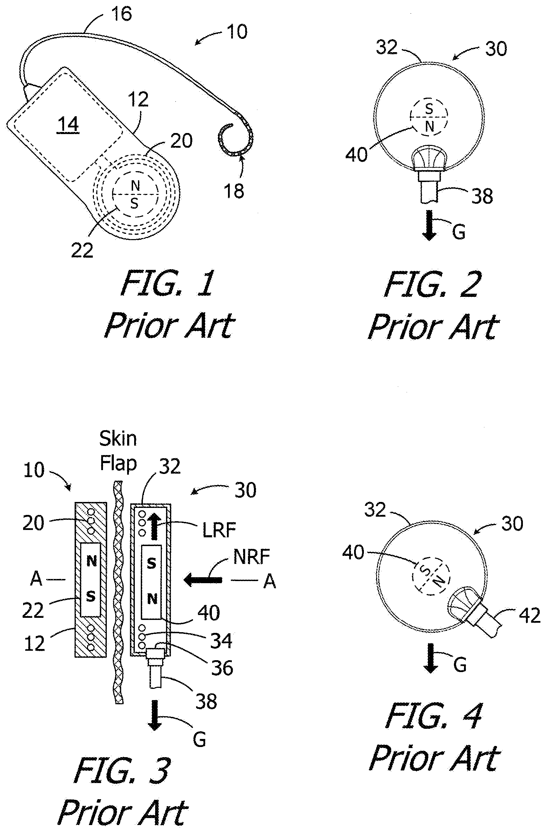

One proposed method of accommodating an MRI magnetic field involves the use of a magnet apparatus with a diametrically magnetized disk-shaped magnet that is rotatable relative to the remainder of the implant about an axis, and that has a N-S orientation which is perpendicular to the axis. U.S. Pat. No. 8,634,909 ("the '909 patent"), for example, discloses a cochlear implant system with a diametrically magnetized and rotatable disk-shaped implant magnet and a diametrically magnetized disk-shaped headpiece magnet. This type of cochlear implant is generally represented by reference numeral 10 in FIGS. 1-3. The cochlear implant 10 includes a flexible housing 12 formed from a silicone elastomer or other suitable material, a stimulation processor 14, a cochlear lead 16 with an electrode array 18, and an antenna 20 that may be used to receive data and power by way of an external antenna. A diametrically magnetized disk-shaped magnet 22 with N and S poles that is rotatable about the axis A relative to the remainder of implant 10 is positioned within the antenna portion of the housing 12. The magnet 22 will rotate about the axis A into alignment with an MRI magnetic that is perpendicular to the axis A.

Other exemplary cochlear implants are provided with a magnet apparatus (not shown) that is better able to rotate into alignment with an MRI magnetic field than a disk-shaped magnet. Here, a plurality of elongate diametrically magnetized magnet are positioned within a frame. The frame and magnets are located within a magnet case. The frame is rotatable relative to the case about the central axis of the case, and the magnets are rotatable relative to the frame about respective axes that are either perpendicular to the central axis or slightly non-perpendicular to the central axis. The magnets align with one another in the N-S direction in the absence of a relatively strong external magnetic field (e.g., an MRI magnetic field), and the at rest N-S orientation of the magnets will be perpendicular to the central axis. Various examples of such magnet apparatus are disclosed in U.S. Pat. No. 10,463,849.

The cochlear implant 10 (or cochlear implants with the magnet apparatus described in the preceding paragraph) may be used in conjunction with a headpiece 30 that includes a housing 32 in which components, such as a printed circuit board (not shown) that carries an antenna 34 and other electronic components, are located. An electrical connector 36 connects the circuit board to a sound processor (e.g., a BTE sound processor) by way of a cable 38. A diametrically magnetized disk-shaped magnet 40 is also provided. The magnetic attraction between the magnets 22 and 40 maintains the position of the headpiece 30 against the skin flap over the cochlear implant 10, and causes the N and S poles of the rotatable implant magnet 22 to align with the S and N poles of the headpiece magnet 40 in the manner shown (or the S and N poles of the magnets in the above-described magnet apparatus). The '909 patent indicates that the headpiece magnet may either be fixed within the headpiece to prevent its rotation, or allowed to rotate on its axis like the implant magnet.

The present inventors have determined that there are a number of issues associated with the above-described cochlear implant systems. For example, the proper retention of the headpiece 30 depends on the normal retention force NRF and the lateral retention force LRF (FIG. 3). The normal retention force NRF is a function of the strength of the diametrically magnetized implant and headpiece magnets 22 and 40 (or multi-magnet magnet apparatus) as well as the distance between the implant and headpiece magnets, while the lateral retention force LRF is a function of the normal retention force NRF and the coefficient of friction between the headpiece and the associated head surface. Pressure on the skin flap can result in discomfort and tissue necrosis when the normal retention force NRF is too high, while the headpiece will not be retained when the normal retention force NRF is too low. Additionally, the normal retention force NRF is maximized when the N and S poles of the implant and headpiece magnets are aligned N to S and S to N and, for a given normal retention force NRF, the lateral retention force LRF is maximized when the N-S direction (or "axis") of the magnets is aligned with the gravitational direction G.

Given that headpieces are typically worn with the headpiece cable extending downwardly in the gravitational direction G (FIG. 3), some conventional headpieces fixedly align the N-S direction of the headpiece magnet with the headpiece cable, thereby typically aligning the N-S direction of the headpiece magnet with the gravitational direction G. The present inventors have determined this can be problematic for persons who do not wear their headpiece in the typical manner and instead wear the headpiece in, for example, the manner illustrated in FIG. 4. Although the strength of the headpiece magnet 44 will cause the rotatable implant magnet 22 (FIG. 3) to rotate into N-S alignment with the headpiece magnet, the N-S direction of the magnets will not be aligned with the gravitational direction G due to the fixed orientation of the headpiece magnet. Such misalignment results in a less than optimal lateral retention force LRF for a given normal retention force NRF. WO2019083540A1 relates generally to implantable cochlear stimulation systems.

The present inventors have also determined that there are many instances where is it difficult to achieve sufficient normal retention force NRF due to, for example, relatively thick patient skin flaps. Given that the magnetic attraction between two magnets is inversely proportional to the square of the distance between the magnets, small changes in the distance between the implant and headpiece magnets can result in a relatively large reduction in the normal retention force NRF and, by extension, the lateral retention force LRF.

### SUMMARY

A cochlear implant headpiece in accordance with the present invention is defined in claim 1 and comprises a housing including a top wall, a bottom wall and a receptacle that extends from the top wall to the bottom wall, that defines an open top end, an open bottom end and a central axis, and that includes a receptacle lock member, a magnet apparatus defining a bottom and including a magnet and a magnet apparatus lock member, and a headpiece antenna on or within the housing. The respective configurations of the receptacle and the magnet apparatus are such that the magnet apparatus can be inserted into the receptacle and, when fully inserted into the receptacle, the magnet apparatus bottom is located within or downwardly beyond the open bottom end of the receptacle. The respective configurations of the receptacle lock member and the magnet apparatus lock member are such that the fully inserted magnet apparatus will be fixed in one of a plurality of rotational orientations around the central axis. The present inventions also include cochlear stimulation systems with a sound processor and/or a cochlear implant in combination with such a headpiece.

There are a variety of advantages associated with such headpieces, systems and kits. By way of example, but not limitation, the N-S orientation of the headpiece magnet relative to the remainder of the headpiece may be adjusted, while reducing the distance between the headpiece magnet and the implanted cochlear implant magnet, thereby increasing in the magnetic attraction between the magnets, as compared to that achieved with conventional headpieces. The kits may include a plurality magnet apparats having different strengths to be tried during the fitting process.

The above described and many other features of the present inventions will become apparent as the inventions become better understood by reference to the following detailed description when considered in conjunction with the accompanying drawings.

### BRIEF DESCRIPTION OF THE DRAWINGS

Detailed descriptions of the exemplary embodiments will be made with reference to the accompanying drawings.
FIG. 1 is a plan view of a conventional cochlear implant.
FIG. 2 is a plan view of a conventional headpiece.
FIG. 3 is a simplified side, section view of a cochlear implant and the headpiece illustrated in FIGS. 1 and 2.
FIG. 4 is a plan view of the headpiece illustrated in FIG. 2.
FIG. 5 is a perspective view of a headpiece in accordance with one embodiment of a present invention.
FIG. 6 is an exploded perspective view of the headpiece illustrated in FIG. 5.
FIG. 7 is a perspective view of a portion of the headpiece illustrated in FIG. 5.
FIG. 8 is a perspective view of a portion of the headpiece illustrated in FIG. 5.
FIG. 9 is a perspective view of a portion of the headpiece illustrated in FIG. 5.
FIG. 10 is a plan view of a portion of the headpiece illustrated in FIG. 5.
FIG. 11 is a perspective view of a portion of the headpiece illustrated in FIG. 5.
FIG. 12 is a perspective view of the magnet apparatus in the headpiece illustrated in FIG. 5.
FIG. 13 is a section view taken along line 13-13 in FIG. 12.
FIG. 14 is an exploded end view of the headpiece illustrated in FIG. 5.
FIG. 15 is a section view of a portion of the headpiece illustrated in FIG. 5.
FIG. 16A-16C are plan views of the headpiece illustrated in FIG. 5 with the magnet apparatus in a single orientation and the remainder of the headpiece in various orientations.
FIG. 17A-17C are plan views of the headpiece illustrated in FIG. 5 with the magnet apparatus and the remainder of the headpiece in various orientations.
FIG. 18 is a section view of a portion of the headpiece illustrated in FIG. 5.
FIGS. 19A and 19B are section and bottom views of the magnet apparatus of the headpiece illustrated in FIG. 5.
FIGS. 20A and 20B are section and bottom views of a magnet apparatus in accordance with one embodiment of a present invention.
FIGS. 21A and 21B are section and bottom views of a magnet apparatus in accordance with one embodiment of a present invention.
FIGS. 22A and 22B are section and bottom views of a magnet apparatus in accordance with one embodiment of a present invention.
FIG. 23 is a perspective view of a magnet apparatus in accordance with one embodiment of a present invention.
FIG. 24 is a section view taken along line 24-24 in FIG. 23.
FIG. 25 is a side view of a headpiece in accordance with one embodiment of a present invention.
FIG. 26 is a side view of a portion of the headpiece illustrated in FIG. 25.
FIG. 27 is an exploded perspective view of a portion of the headpiece illustrated in FIG. 25.
FIG. 28 is a section view of a portion of the headpiece illustrated in FIG. 25.
FIG. 29 is a section view of a magnet apparatus in accordance with one embodiment of a present invention.
FIG. 30 is a section view of a magnet apparatus in accordance with one embodiment of a present invention.
FIG. 31 is a section view of a portion of a headpiece in accordance with one embodiment of a present invention.
FIG. 32 is a perspective view of a magnet apparatus in accordance with one embodiment of a present invention.
FIG. 33 is a section view taken along line 33-33 in FIG. 32.
FIG. 34 is a section view of a magnet apparatus in accordance with one embodiment of a present invention.
FIG. 35 is a section view of a magnet apparatus in accordance with one embodiment of a present invention.
FIG. 36 is a plan view of a kit in accordance with one embodiment of a present invention.
FIG. 37 is a block diagram of an ICS system in accordance with one embodiment of a present invention.
FIG. 38 is a block diagram of a headpiece in accordance with one embodiment of a present invention.

### DETAILED DESCRIPTION OF THE EXEMPLARY EMBODIMENTS

The following is a detailed description of the best presently known modes of carrying out the inventions. This description is not to be taken in a limiting sense, but is made merely for the purpose of illustrating the general principles of the inventions.

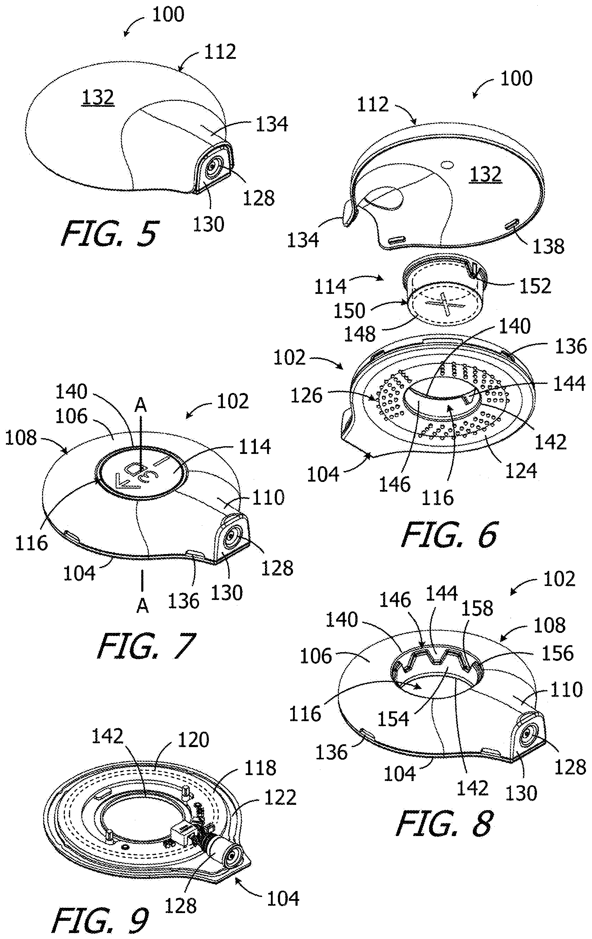

An exemplary headpiece in accordance with at least one of the present inventions is illustrated in FIGS. 5-9 and is generally represented by reference numeral 100. The exemplary headpiece 100 may include a housing 102, with a base member 104 and a dome-like member 106 that together define a ring-like portion 108 and a projection 110, and a removable cap 112 that may be secured to the housing. A removable diametrically magnetized headpiece magnet apparatus (or "magnet apparatus") 114 is located within a receptacle 116 that extends through the housing 102. As is discussed in greater detail below, the magnet apparatus 114 may be fixed at any one of a plurality of rotational orientations located around a central axis A (of both the magnet apparatus 114 and the receptacle 116) to obtain the desired N-S direction of the magnet apparatus relative to the housing 102. The magnet apparatus 114 may also be removed and replaced with another magnet apparatus having different properties such as, for example, greater magnetic strength.

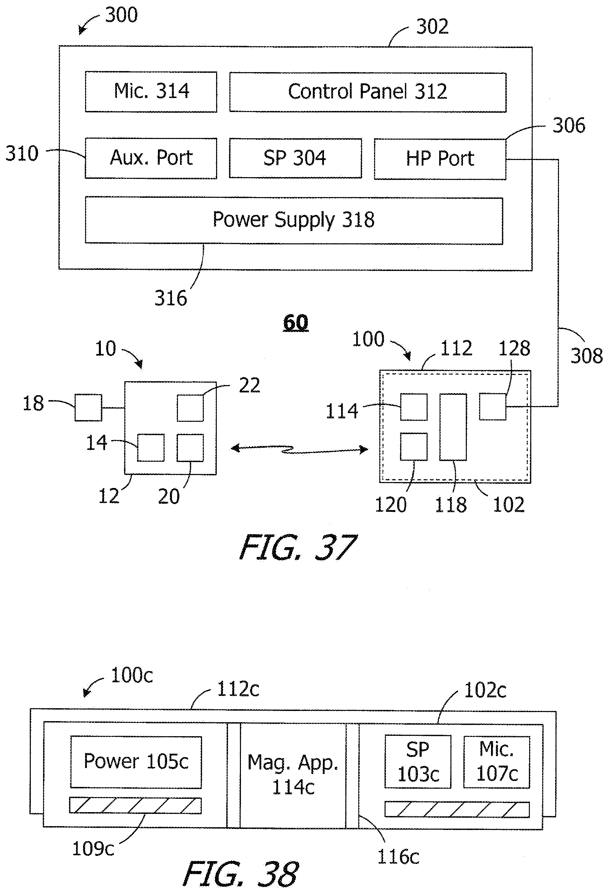

An annular printed circuit board (PCB) 118 that carries various headpiece electronic components on the top side thereof is located within the housing ring-like portion 108. An antenna may be may be located on, located within, or otherwise carried by the housing 102. In the illustrated embodiment, an antenna 120 is located on the bottom side of the PCB 118 and the PCB is positioned on the top (or "interior") surface 122 of the base member 104. In other implementations, the antenna may be carried by the base member 104. In the illustrated embodiment, the PCB 118 and antenna 120 are hermetically sealed within the housing, and the dome-like member 106 may be secured to the base member 104 through the use of ultrasonic welding, adhesive, or any other suitable instrumentality. The bottom (or "exterior") surface 124 of the base member 104 may be concave or flat, and may include a plurality of protrusions 126. A connector 128, such as a RF connector, is connected to the PCB 118 and extends through an end wall 130 on the housing projection 110. The connector 128 may be used to connect the PCB 118 to a sound processor (e.g., a BTE sound processor) by way of a cable 308 (FIG. 37). The exemplary cap 112 has a main portion 132 that covers the magnet apparatus 114 and receptacle 116, and a hood 134 that covers the projection 110. The housing 102 and cap 112 may be attached to one another with any suitable instrumentality. In the illustrated implementation, the housing 102 includes a plurality of latch indentations 136 that are engaged by a corresponding plurality of latches 138 on the cap 112 when the cap is positioned over the housing 102 in the manner illustrated in FIG. 5.

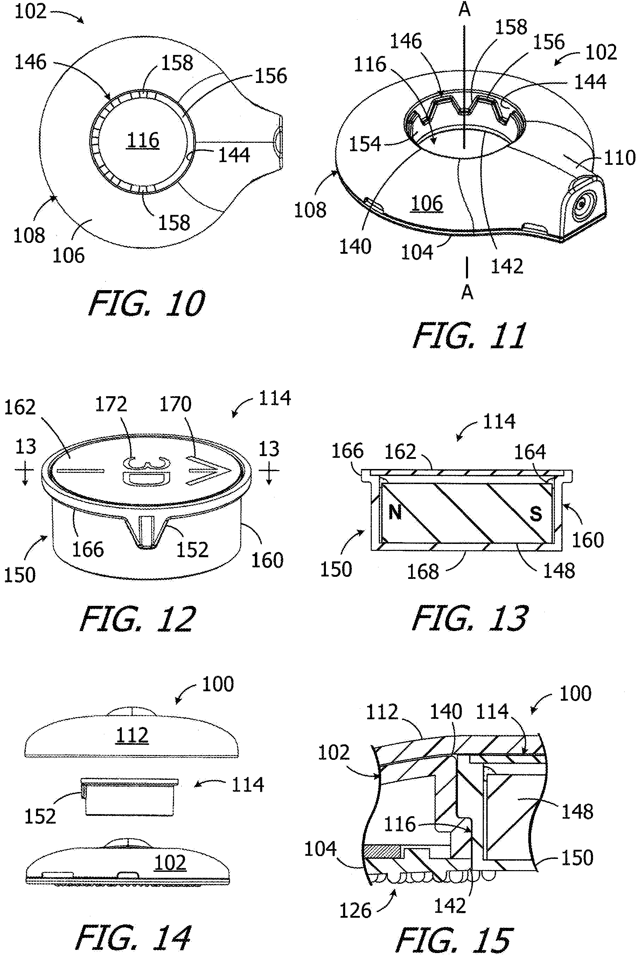

Referring more specifically to FIGS. 6-8, the exemplary receptacle 116 extends completely through the housing 102, has an open top end 140 and an open bottom end 142, and includes a cylindrical wall 144 and a lock member 146 on the cylindrical wall. It should be noted here that, in the context of the present application, words such as "bottom" and "downwardly" are used to describe structures that are adjacent to or that face the wearer's head when the headpiece is in use, while words such as "top" and "upwardly" are used to describe structures that are not adjacent to or that face away from the wearer's head when the headpiece is in use. The exemplary magnet apparatus 114 includes a diametrically magnetized magnet 148 (FIG. 6) that is located within a magnet case 150. A lock member 152 is located on, and projects outwardly from, the outer surface of the magnet case 150. The lock members 146 and 152 cooperate with one another to fix the rotational orientation of the magnet apparatus 114 relative to the housing 102 to the orientation that provides the desired N-S direction of the magnet apparatus relative to the housing. To that end, and turning to FIGS. 10 and 11, the exemplary lock member 146 includes a base 154 that defines a top surface 156 and a plurality of indentations 158 extending downwardly from the top surface. There are five indentations 158 in the illustrated embodiment. Two of the indentations 158 are angularly offset from one another by 180 degrees around the axis A, and the remaining three indentations are located between the two that are offset by 180 degrees. The indentations 158, which may be V-shaped (as shown) or any other suitable shape, are also equidistant (45 degrees) from one another in the illustrated implementation.

As illustrated in FIGS. 12 and 13, the magnet case 150 of the exemplary magnet apparatus 114 includes a cylindrical container 160 that is configured to receive the diametrically magnetized magnet 148 and a cover 162 that prevents removal of the magnet from the magnet case. The cover 162 may also be used to hermetically seal the magnet 148 within the container 160. The cover 162 may be secured to the container 160 through the use of ultrasonic welding, adhesive, or any other suitable instrumentality. In other implementations, the magnet case may be a one-piece structure in which the magnet is molded. The magnet 148 may in some instances also be secured in container 160 with adhesive 164. The container 160 is sized and shaped such that it can be placed into the receptacle 116. A flange 166 is provided at the top end of the container 160, and the lock member 152 extends downwardly from the flange. The magnet apparatus flange 166 extends radially outward from the container 160. The flange 166 also rests on the lock member top surface 156 when the magnet apparatus is fully inserted to receptacle 116 and, as can be seen in FIG. 7, the respective sizes of the magnet apparatus 114 and the receptacle 116 result in the top surfaces of the cover 162 and the flange 166 being flush with the adjacent top surface of the housing annular portion 108.

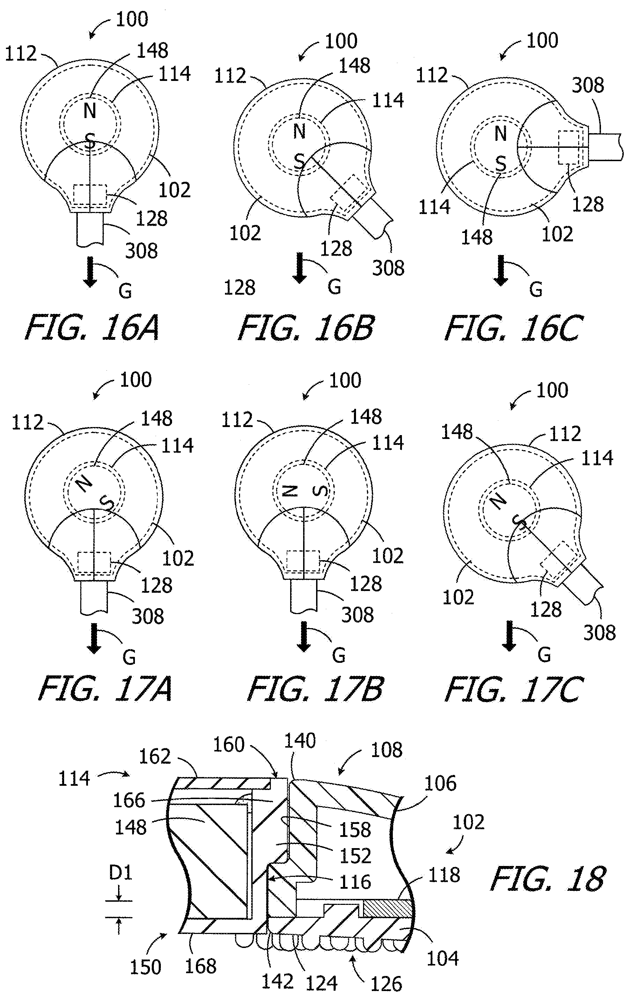

As is discussed in greater detail below with reference to FIG. 18, the respective configurations of the magnet apparatus 114 and the receptacle 116 result in the container bottom wall 168 extending to or through the open bottom end 142 of the receptacle 116, and this is the same regardless of which indentation 158 the lock member 152 is located within. Put another way, the position of the magnet apparatus 114 along the central axis A is the same regardless of the rotational orientation of magnet apparatus 114 around the central axis A. As a result, the distance between the headpiece magnet 148 and the implant magnet are decreased, and the attraction force between the magnets is correspondingly increased, as compared to an arrangement where the bottom wall of the magnet apparatus container rests on top (or "inner") surface of the headpiece bottom wall.

The lock member 152 is configured to fit into the indentations 158. In the illustrated implementation, the lock member 152 is V-shaped. Other suitable magnet apparatus lock member configurations, which correspond to other housing lock member configurations, may be employed. By way of example, but not limitation, the magnet apparatus lock member and the housing indentations may be rectangular or semi-circular. Alternatively, or in addition, the magnet apparatus lock member may be in the form of an indentation that extends upwardly from the container bottom wall 168, while the housing lock member may include corresponding projections in place of the illustrated indentations.

It should also be noted that the lock member 152 and/or the flange 166 may cooperate with the lock member 146 to prevent the magnet apparatus 114 from exiting the housing 102 by way of the open bottom end 142 of the receptacle 116. In the illustrated embodiment, once the magnet apparatus 114 is fully inserted into the receptacle 116, the lock member 152 will be unable to pass the associated indentation 158, and the flange 166 (which has a diameter greater than that of the lock member 146) will abut the base top surface 156, thereby preventing further downward movement.

The removable cap 112 prevents the magnet apparatus 114 from exiting the housing 102 by way of the open top end 140 of the receptacle 116. Referring to FIGS. 14 and 15, the magnet apparatus 114 may be placed into the receptacle 116 in the desired N-S orientation while the cap 112 is disconnected from the housing 102. The cap 112 may then be placed onto the housing 102 and secured thereto with, for example, the illustrated indentations 136 and latches 138 (FIG. 6) to maintain the desired N-S orientation and hold the magnet apparatus 114 within the receptacle 166.

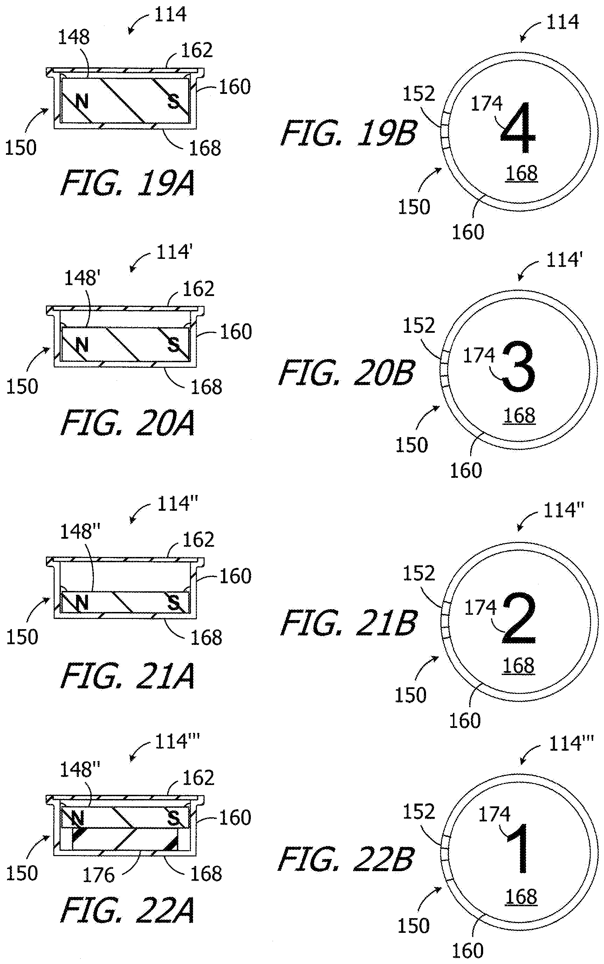

The magnet apparatus 114 may also include visible and/or tactile indicia (or some other instrumentality) which identifies the N-S direction of the diametrically magnetized magnet 148 and, in some instances, provides other information about the magnet apparatus 114. Such information allows the user to, for example, position the magnet apparatus 114 within the receptacle 116 in the desired N-S orientation. The exemplary magnet apparatus 114 includes indicia on the cover 162 in the form of an arrow 170 that identifies the N-S direction and one or more alpha and/or numeric elements 172 which indicate the type of magnet (i.e., diametrically magnetized or axially magnetized) that is located within the magnet case 150, as is shown in FIG. 12. Such information may be combined into a single indicia type that provides more than one type of information. For example, an arrow could be used to represent that the magnet is a diametrically magnetized magnet as well as the N-S direction of the magnet. Another example involves the use of one or more chevrons. Here, the orientation of the chevrons may be used to identify the N-S direction of the magnet, while the number of chevrons may be used to indicate the strength of the magnet apparatus. Color, such the color of the container 160 and/or cover 162, may also be employed. Other indicia may be used to identify the magnetic strength of a magnet apparatus, as is discussed below with reference to FIGS. 19A-22B.

The present headpiece 100 (as well as the other headpieces described herein) may be arranged one the wearer's head in a variety of orientations while, at the same time, the magnet apparatus 114 (as well as the other magnet apparatus described herein) may be oriented in such a manner that the N-S direction of the magnet is aligned with the gravitation direction or, if so desired, the N-S direction of the magnet is not aligned with the gravitation direction. For example, as illustrated in FIGS. 16A-16C, the headpiece 100 may be oriented in such a manner that the lateral retention force is maximized for a particular magnet strength. The headpiece 100 may oriented such that the connector 128 faces in the gravitational direction G and the cable 308 extends in the gravitational direction G (FIG. 16A), or such that the connector 128 faces in a direction that is offset from the gravitational direction G by 45 degrees and the cable 308 extends in a direction that is offset from the gravitational direction G by 45 degrees (FIG. 16B), or such that the connector 128 faces in a direction that is offset from the gravitational direction G by 90 degrees and the cable 308 extends in a direction that is offset from the gravitational direction G by 90 degrees (FIG. 16C). In each instance, the magnet apparatus 114 is fixed within the housing 102 in an orientation that results in the N-S direction of the diametrically magnetized magnet 148 being the gravitational direction G, thereby maximizing the lateral retention force. In other instances, the lateral retention force may be reduced by orienting the magnet apparatus 114 within the housing 102 in such a manner that N-S direction of the diametrically magnetized magnet 148 is in a direction other than the gravitational direction G. For example, the headpiece 100 may oriented such that the connector 128 faces in the gravitational direction G and the cable 308 extends in the gravitational direction G, while the magnet apparatus 114 is oriented within the housing 102 in such a manner that N-S direction of the diametrically magnetized magnet 148 is offset from the gravitational direction G by 45 degrees (FIG. 17A) or by 90 degrees (FIG. 17B). The headpiece 100 may also be oriented such that the connector 128 faces in a direction that is offset from the gravitational direction G by 45 degrees and the cable 308 extends in a direction that is offset from the gravitational direction G by 45 degrees, while the magnet apparatus 114 is oriented within the housing 102 in such a manner that N-S direction of the diametrically magnetized magnet 148 is also offset from the gravitational direction G by 45 degrees (FIG. 17C).

Turning to FIG. 18, and as alluded to above, when the magnet apparatus 114 is fully inserted into the receptacle 116 in the exemplary implementation, with the lock member 152 in one of the lock member indentations 158, the case bottom wall 168 extends through the housing base member 104 to the receptacle open bottom end 142. In this instance, the bottom end of the case 150 is aligned with the adjacent portion of the bottom surface 124 of the base member 104. As compared to an otherwise identical headpiece where the receptacle does not extend though the bottom wall of the housing (because the bottom end is not open) and the bottom wall of the magnet apparatus container rests on the inner surface of the headpiece bottom wall, the distance between the present headpiece magnet 148 and the implanted cochlear implant magnet (not shown) will be decreased by a distance D1. The distance D1 is equal to the thickness of the base member 104 in the illustrated embodiment. Because the magnetic attraction between two magnets is inversely proportional to the square of the distance between the magnets, the percent increase in the magnetic attraction is relatively large even when distance D1 is a small distance. Assuming for example that a magnet-to-magnet distance of 6.0 mm is decreased by 0.6 mm, the magnetic attraction will be 12% greater.

Magnet apparatus with different magnetic strengths may be used in conjunction with the same headpiece housing 102. In the illustrated implementation, where the magnets within the magnet apparatus are formed from the same magnetic material (e.g., N55 grade neodymium) and the case bottom wall 168 always faces the implant magnet, the size of a magnet and the distance of the magnet from the case bottom wall will be determinative of magnet apparatus strength. Each magnet apparatus may be provided with indicia (or another suitable instrumentality) that allows the user to discern the relative magnetic strength of that magnet apparatus.

Referring first to FIGS. 19A and 19B, the above-described magnet apparatus 114 may be provided with indicia 174 on the container bottom wall 168 that is representative of relative magnetic strength. The magnet apparatus illustrated in FIGS. 20A-22B are substantially similar to magnet apparatus 114 and similar elements are represented by similar reference numerals. For example, the magnet cases 150 are the same size and shape. The respective magnetic strengths of the magnet apparatus illustrated in FIGS. 20A-22B are, however, different than that of magnet apparatus 114 and are different than those of one another. The exemplary magnet apparatus 114' illustrated in FIGS. 20A-20B includes a diametrically magnetized magnet 148' that, like the magnet 148 of magnet apparatus 114, is in contact with the container bottom wall 168 and the bottom wall includes indicia 174. The magnet 148' in magnet apparatus 114' is, however, smaller than the magnet 148 in magnet apparatus 114. As such, the strength of magnet apparatus 114 is greater than that of magnet apparatus 114'. Similarly, the exemplary magnet apparatus 114" illustrated in FIGS. 21A-21B includes a diametrically magnetized magnet 148" that, like the magnets 148 and 148' of magnet apparatus 114 and 114', is in contact with the container bottom wall 168 and the bottom wall includes indicia 174. The magnet 148" in magnet apparatus 114" is, however, smaller than the magnet 148' in magnet apparatus 114'. As such, the strength of magnet apparatus 114' is greater than that of magnet apparatus 114". Turning to FIGS. 22A and 22B, the magnet apparatus 114‴ is identical to magnet apparatus 114" but for the fact that the magnet 148" in magnet apparatus 114‴ is separated from the container bottom wall 168 by a non-magnetic spacer 176 (e.g., a polyoxymethylene spacer). The distance between the magnet 148" in magnet apparatus 114‴ and the implanted cochlear implant magnet will be greater than the distance associated with of the magnet apparatus 114" and, accordingly, the strength of the magnet apparatus 114" is considered to be greater than that of the magnet apparatus 114‴.

In other implementations, the variations in magnet apparatus strength may be accomplished through variations in magnet strength. For example, the magnets 148', 148" and 148‴ may be the same size as the magnet 148, and occupy the same position within the associated magnet apparatus, but be formed from successively weaker magnetic materials.

The indicia 174 of the magnet apparatus 114-114‴ is indicative of relative strength. In the illustrated implementation, numeric indicia is employed with the numbers increasing from weakest to strongest. For example, magnet apparatus 114‴ is labeled "1" and magnet apparatus 114 is labeled "4."

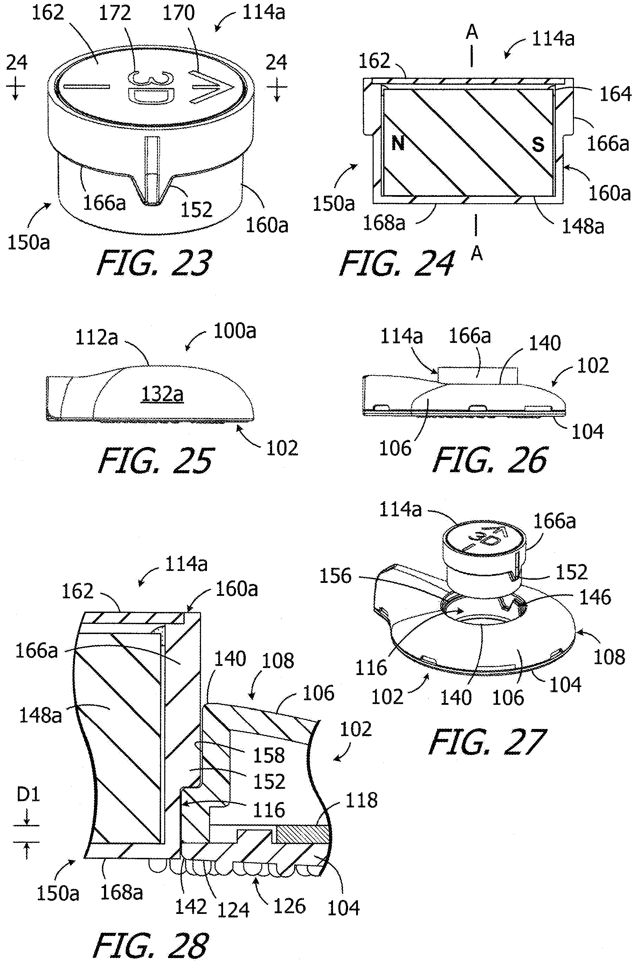

Larger magnets (as compared to magnet 148) may be used to create still stronger magnet apparatus. Referring to FIGS. 23 and 24, the exemplary magnet apparatus 114a is substantially similar to magnet apparatus 114 and similar elements are represented by similar reference numerals. For example, the magnet apparatus 114a includes a diametrically magnetized magnet 148a and a magnet case 150a. The magnet case 150a has container 160a that is configured to receive the magnet 148a, a cover 162 that prevents removal of the magnet from the magnet case, adhesive 164, and a flange 166a at the top end of the container 160a. A lock member 152 is located on, and projects outwardly from, the outer surface of the magnet case 150a. The magnet 148a is, however, larger than magnet 148. Although the diameters of the magnets are the same (e.g., 11 mm), the magnet 148a is longer in the axial direction than the magnet 148. Similarly, although the inner and outer diameters of the magnet cases 150 and 150a are the same, magnet case 150a is longer in the axial direction than magnet case 150 to accommodate the longer magnet 148a and, in particular, the container 160a is longer than the container 160. The magnet case 150a is also sized, shaped and otherwise configured such that the magnet apparatus 114a can be placed into the receptacle 116 of housing 102, and the distance between the lock member 152 and the container bottom wall 168a is the same as that of the magnet apparatus 114. As such, the magnet apparatus 114a may also be fixed in the desired N-S orientation in the manner described above with reference to FIGS. 10-18.

Turning to FIGS. 25-27, the exemplary headpiece 100a with magnet apparatus 114a is essentially identical to headpiece 100 with magnet apparatus 114, and similar elements are represented by similar reference numerals. The exemplary headpiece 100a includes the housing 102 (as well as the components therein), the larger magnet apparatus 114a, and a removable cap 112a that is configured to accommodate the larger magnet apparatus. When the magnet apparatus 114a is fully inserted into the housing receptacle 116, with the respective lock members 146 and 152 engaged (i.e., with the lock member 152 in one of the indentations 158) and the flange 166a resting on the base top surface 156, a portion of the flange will extend upwardly beyond the receptacle top end 140 (FIG. 26). The removable cap 112a accommodates the larger magnet apparatus 114a by way of a bulbous main portion 132a that rests on the magnet apparatus when the cap is secured to the housing 102 by way of the latch indentations 136 and latches 138 or other suitable instrumentalities.

As is illustrated for example in FIG. 28, the case bottom wall 168a of the magnet apparatus 114a extends through the housing base member 104 to the receptacle open bottom end 142, and the bottom end of the case 150a is aligned with the adjacent portion of the bottom surface 124 of the base member, when the magnet apparatus is fully inserted into the receptacle 116. Such an arrangement reduces the distance between headpiece magnet 148a and the implanted cochlear implant magnet (not shown) by a distance D1, as compared to an arrangement where the magnet apparatus rests on the inner surface of the housing base member, and increases the magnetic attraction between the magnets, as is discussed in greater detail above with reference to FIG. 18.

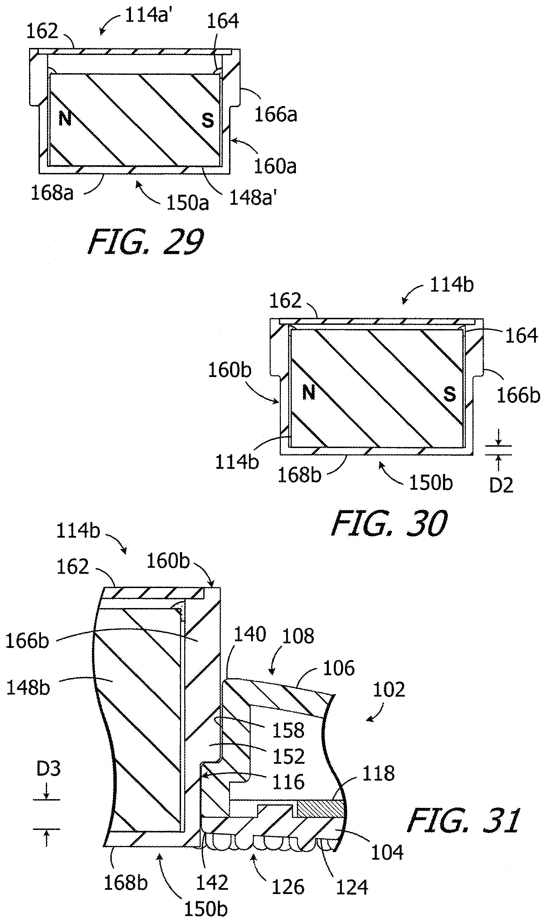

Here too, the size of a magnet and/or the distance of the magnet from the case bottom wall 168a may be varied. For example, and referring to FIG. 29, the exemplary magnet apparatus 114a' is essentially identical to magnet apparatus 114a but for a smaller diametrically magnetized magnet 148a'. The diameters of the exemplary magnets are the same, but the magnet 148a' is shorter in the axial direction than the magnet 148. The strength of magnet apparatus 114a' is, accordingly, less than that of magnet apparatus 114a.

The exemplary magnet apparatus 114b illustrated in FIGS. 30 and 31, on the other hand, is configured to place the associated magnet closer to the implant magnet than the magnet apparatus 114a. The magnet apparatus 114b is substantially similar to the magnet apparatus 114a and similar elements are represented by similar reference numerals. For example, the magnet apparatus 114b includes a diametrically magnetized magnet 148b and a magnet case 150b. The magnet case 150b has container 160b that is configured to receive the diametrically magnetized magnet 148b, a cover 162 that prevents removal of the magnet from the magnet case, adhesive 164, and a flange 166b at the top end of the container 160b. A lock member 152 is also provided. The magnet apparatus 114b is, however, configured such that the distance between the bottom end of the flange 166b and the outer surface of the container bottom wall 168b is greater than that of the magnet apparatus 114 by a distance D2. The distance D2 in the illustrated embodiment is slightly less or equal to the length of the protrusions 126 measured from the free ends thereof to the base member 104 (e.g., about 0.5 mm). The additional length of this portion of the magnet case 150b results in the magnet apparatus 114b extending through and downwardly beyond the adjacent portion of the bottom surface 124 of the base member 104. Such an arrangement decreases the distance between headpiece magnet 148b and the implanted cochlear implant magnet (not shown) by a distance D3 (which is the sum of distances D1 and D2), as compared to an arrangement where the magnet apparatus rests on the inner surface of the housing base member, and increases the magnetic attraction between the magnets.

It should also be noted here that the present headpieces may also include magnet apparatus with axially magnetized magnets for use in conjunction with certain cochlear implants such as, for example, cochlear implants that include an axially magnetized implant magnet. More specifically, the housing 102 (as well as the internal components thereof) may be used in conjunction with a magnet apparatus, including an axially magnetized magnet, that is configured to be received within the housing receptacle 116. This facilitates production of a modular headpiece which may be used with cochlear implants that require an axially magnetized headpiece magnet as well as with cochlear implants that require that required a diametrically magnetized headpiece magnet. Although the rotational orientation of an axially magnetized magnet has no effect on the strength of the attraction between the headpiece magnet and the cochlear implant magnet, magnet apparatus with axially magnetized magnets may include the same magnet case that is employed in magnet apparatus with diametrically magnetized (e.g., magnet case 150) in order to simplify the manufacturing, inventory, and distribution processes. Alternatively, otherwise identical magnet cases without the lock member 152 may be provided.

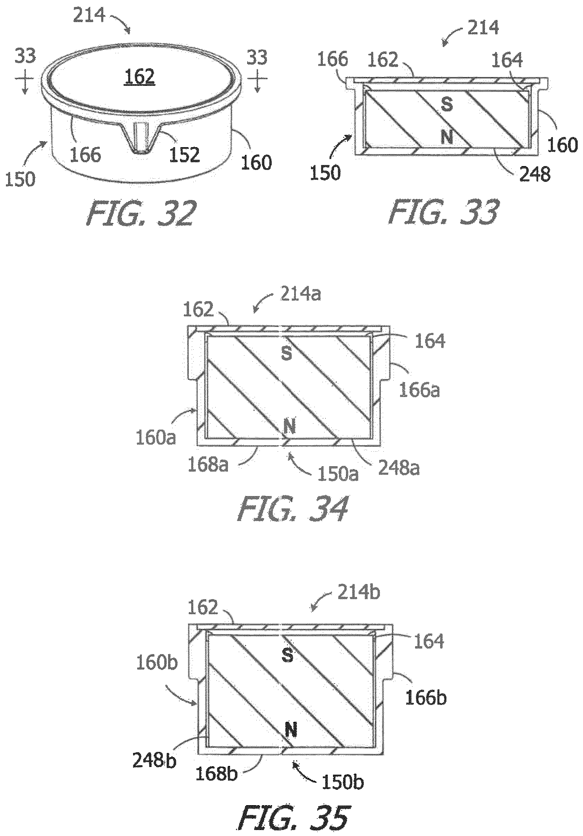

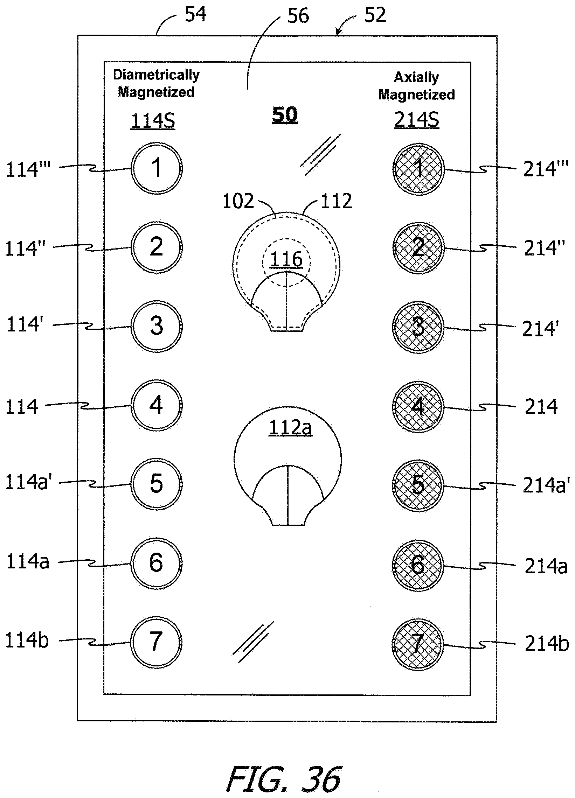

Referring to FIGS. 32 and 33, the exemplary magnet apparatus 214 illustrated therein is substantially similar to the magnet apparatus 114 described above with reference to FIGS. 12 and 13. Here, however, the magnet apparatus 214 includes an axially magnetized magnet 248 in the above-described magnet case 150 and lacks indicia representative N-S direction. Other indicia may be provided in some instances to identify the magnet apparatus 214 as an axially magnetized magnet apparatus. The exemplary magnet apparatus 214 is configured to be received within the housing receptacle 116 in the same manner as the magnet apparatus 114. The strength of the magnet apparatus 214 may be modified in the manner described above with reference to FIGS. 19A-22B. In particular, the magnet apparatus 214', 214" and 214‴ (shown in FIG. 36) may, but for the use of an axially magnetized magnet, be configured in the same manner as the magnet apparatus 114', 114" and 114"'. As such, the strength of magnet apparatus 214 is greater than that of magnet apparatus 214', the strength of magnet apparatus 214' is greater than that of magnet apparatus 214", and the strength of magnet apparatus 214" is greater than that of magnet apparatus 214‴.

Turning to FIG. 34, magnet apparatus 214a illustrated therein is substantially similar to the magnet apparatus 114a described above with reference to FIGS. 23-28. Here, however, the magnet apparatus 214a includes an axially magnetized magnet 248a in the above-described magnet case 150a and lacks indicia representative N-S direction. Other indicia may be provided in some instances to identify the magnet apparatus 214a as an axially magnetized magnet apparatus. The exemplary magnet apparatus 214a is configured to be received within the housing receptacle 116 in the same manner as the magnet apparatus 114a. The strength of the magnet apparatus 214a may be modified in the manner described above with reference to FIG. 29, i.e. by reducing the size of the magnet, which results in a magnet apparatus 214a' (shown in FIG. 36) with a magnetic strength that is less than that of magnet apparatus 214a.

The exemplary magnet apparatus 214b illustrated in FIG. 35 is substantially similar to the magnet apparatus 114b described above with reference to FIGS. 30 and 31. Here, however, the magnet apparatus 214b includes an axially magnetized magnet 248b in the above-described magnet case 150b and lacks indicia representative N-S direction. Other indicia may be provided in some instances to identify the magnet apparatus 214b as an axially magnetized magnet apparatus. The exemplary magnet apparatus 214b is configured to be received within the housing receptacle 116 in the same manner as the magnet apparatus 114b.

Magnet apparatus such as those described above may form part of magnet apparatus systems that allow audiologists to select the most appropriate magnet apparatus strength (and magnet apparatus orientation) during the fitting process. As illustrated for example in FIG. 36, the exemplary magnet apparatus system 114S may include the diametrically magnetized magnet apparatus 114‴, 114", 114', 114, 114a', 114a and 114b with indicia in the form of numerals 1-7 on the bottom surfaces thereof. The numerals identify in ascending order the relative strengths of the magnet apparatus in the magnet apparatus system 114S. Similarly, the exemplary magnet apparatus system 214S may include the axially magnetized magnet apparatus 214‴, 214", 214', 214, 214a', 214a and 214b with indicia in the form of numerals 1-7 on the bottom surfaces thereof. The numerals identify in ascending order the relative strengths of the magnet apparatus in the magnet apparatus system 214S. The bottom surfaces of magnet apparatus in the magnet apparatus system 214S includes other indicia such as color (as shown) to indicate that the magnet apparatus are axially magnetized. The magnet apparatus systems 114S and 214S may also be combined and considered a single magnet apparatus system.

One or both of the exemplary magnet apparatus systems 114S and 214S may be provided as part of a kit 50 to, for example, audiologists for use during the fitting process. Here, the magnet apparatus systems 114S and 214S may be located within the packaging 52, which in the illustrated implementation includes a box or other enclosure 54 with a cover 56, for shipping and storage. The cover 56 may be transparent, as shown, or opaque. The kit 50 may also include a headpiece housing 102, a cap 112 and a cap 112a.

The exemplary headpiece 100 (or 100a) may be used in ICS systems such as, for example, the exemplary ICS system 60 illustrated in FIG. 37. The system 60 includes the cochlear implant 10, a headpiece 100 (or 100a), and a sound processor 200, such as a body worn sound processor or a behind-the-ear sound processor.

The exemplary sound processor 300 is a body worn sound processor that includes a housing 302 in which and/or on which various components are supported. Such components may include, but are not limited to, sound processor circuitry 304, a headpiece port 306 that may be connected to the headpiece 100 by a cable 308, an auxiliary device port 310 for an auxiliary device such as a mobile phone or a music player, a control panel 312, one or more microphones 314, and a power supply receptacle 316 for a removable battery or other removable power supply 318 (e.g., rechargeable and disposable batteries or other electrochemical cells). The sound processor circuitry 304 converts electrical signals from the microphone 314 into stimulation data.

During use, the magnet of an above-described headpiece magnet apparatus (e.g., magnet 148 of magnet apparatus 114) will be attracted to the implant magnet 22, thereby aligning the headpiece antenna 120 with the implant antenna 20. The stimulation data and, in many instances power, is supplied to the headpiece 100, which transcutaneously transmits the stimulation data, and in many instances power, to the cochlear implant 10 by way of a wireless link between the antennas. In at least some implementations, the cable 308 will be configured for forward telemetry and power signals at 49 MHz and back telemetry signals at 10.7 MHz. It should be noted that, in other implementations, communication between a sound processor and a headpiece and/or auxiliary device may be accomplished through wireless communication techniques.

It should be noted that the present inventions have application in ICS systems which are configured such that all of the external components (e.g., the battery, the microphone, the sound processor, and the antenna) are carried within a single headpiece, and there is no BTE or body worn sound processor connected to the headpiece by a cable. One example of such a headpiece is generally represented by reference numeral 100c in FIG. 38. The exemplary headpiece 100c may include a housing 102c with an internal volume that contains a sound processor 103c, a power supply 105c, a microphone 107c and an annular antenna 109c. The exemplary headpiece 100c also includes a magnet apparatus 114c, a receptacle 116c, and a removable cap 112c that may be secured to the housing. The magnet apparatus 114c and receptacle 116c may have configurations that are the same as, or that are similar to, the configurations of any of the magnet apparatus and receptacles described above. For example, the receptacle 116c, which extends completely though the associated housing in a manner similar to receptacle 116 (FIGS. 6 and 8), has open top and bottom ends. The removable cap 112c holds the magnet apparatus 114c in a manner similar to cap 112 (FIGS. 14 and 15). It should be noted, however, that due to the additional components such as the power supply 105c and sound processor 103c, the headpiece 100c may be heavier than headpieces intended for use with a separate sound processor. As such, the magnet apparatus 114c and receptacle 116c may also be larger than the other magnet apparatus and receptacles described above to accommodate the larger diametrically magnetized and axially magnetized magnets that may be required by the heavier headpiece 100c.

Although the inventions disclosed herein have been described in terms of the preferred embodiments above, numerous modifications and/or additions to the above-described preferred embodiments would be readily apparent to one skilled in the art. By way of example, but not limitation, although the exemplary headpieces 100 and 100a described above do not include microphones, one or more microphones may be provided within the housing 102 in other implementations. The inventions also include any combination of the elements from the various species and embodiments disclosed in the specification that are not already described. It is intended that the scope of the present inventions extend to all such modifications and/or additions and that the scope of the present inventions is limited solely by the claims set forth below.

## Claims

1. A cochlear implant headpiece for use with a cochlear implant, the cochlear implant headpiece comprising:
a housing (102, 102c) includir g a top wall (106), a bottom wall (104) and a receptacle (116, 116c) that extends from the top wall to the bottom wall, that defines an open top end (140), an open bottom end (142) and a central axis (A), and that includes a receptacle lock member (146);
a magnet apparatus (114-114‴, 114a, 114a', 114b, 114c, 214, 214a, 214b) defining a bottom and including a magnet (148-148‴, 148a, 148b, 248, 248a, 248b) and a magnet apparatus lock member (152); and
a headpiece antenna (120) on or within the housing;
wherein
the respective configurations of the receptacle (116, 116c) and the magnet apparatus (114-114‴, 114a, 114c, 214, 214a, 214b) are such that the magnet apparatus can be inserted into the receptacle, and
the respective configurations of the receptacle lock member (146) and the magnet apparatus lock member (152) are such that the fully inserted magnet apparatus will be fixed in one of a plurality of rotational orientations around the central axis (A), **characterised in that** said headpiece is conf gured such that when the magnet apparatus is fully inserted into the receptacle, the magnet apparatus bottom (168, 168a, 168b) is located within or downwardly beyond the open bottom end (142) of the receptacle.

2. A cochlear implant headpiece as claimed in claim 1, wherein
the magnet apparatus (114-114‴, 114a, 114a', 114b, 114c, 214, 214a, 214b) is not rotatable relative to the magnet receptacle (116, 116c) when fully inserted into the receptacle.

3. A cochlear implant headpiece as claimed in claim 1 or claim 2, wherein
when fully inserted into the receptacle (116), the magnet apparatus bottom (168b) is located downwardly beyond the open bottom end (142) of the receptacle.

4. A cochlear implant headpiece as claimed in any one of claims 1 to 3, wherein
the magnet apparatus (114-114‴, 114a, 114a', 114b, 114c, 214, 214a, 214b) includes a case (150, 150a, 150b) and a cover (162) and the magnet (148-148‴, 148a, 148b, 248, 248a, 248b) is located within the case.

5. A cochlear implant headpiece as claimed in any one of claims 1 to 4, wherein
the magnet apparatus (114-114‴, 114a, 114a', 114b, 114c, 214, 214a, 214b) includes a radially extending flange (166, 166a, 166b);
the receptacle lock member (146) defines a top surface (156); and
the respective configurations of the receptacle (116, 116c) and the magnet apparatus are such that the radially extending flange abuts the top surface (156) of the receptacle lock member when the magnet apparatus is fully inserted into the receptacle.

6. A cochlear implant headpiece as claimed in any one of claims 1 to 5, wherein
the receptacle lock member (146) includes a plurality of slots (158); and
the magnet apparatus lock member (152) comprises a projection.

7. A cochlear implant headpiece as claimed in any one of claims 1 to 6, wherein
the housing (102, 102c) defines a ring-like portion (108) and includes a circuitry (118, 103c) within the ring-like portion.

8. A cochlear implant headpiece as claimed in claim 7, wherein
the headpiece antenna (120, 109c) is located within the substantially ring-like portion (108).

9. A cochlear implant headpiece as claimed in any one of claims 1 to 8, wherein
the housing top wall (106) def nes a housing top surface;
the magnet apparatus (114-114"', 214) defines a magnet apparatus top surface; and
the respective configurations of the receptacle (116, 116c) and the magnet apparatus are such that the magnet apparatus top surface is flush with an adjacent portion of the housing top surface when the magnet apparatus is fully inserted into the receptacle.

10. A cochlear implant headpiece as claimed in any one of claims 1 to 8, wherein
the housing top wall (106) defines a housing top surface;
the magnet apparatus (114a, 114a', 114b, 214a, 214b) defines a magnet apparatus top surface; and
the respective configurations of the receptacle (116) and the magnet apparatus are such that the magnet apparatus top surface is located outwardly beyond an adjacent portion of the housing top surface when the magnet apparatus is fully inserted into the receptacle.

11. A cochlear implant headpiece as claimed in any one of claims 1 to 10, wherein
the headpiece magnet (148-148‴, 148a, 148a', 148b) comprises a diametrically magnetized magnet.

12. A cochlear implant headpiece as claimed in any one of claims 1 to 10, wherein
the headpiece magnet (248, 248a, 248b) comprises an axially magnetized magnet.

13. A cochlear implant headpiece as claimed in any one of claims 1 to 12, further comprising:
a cap (112, 112c) configured to be mounted on the housing (102, 102c) and to cover the magnet receptacle (116, 116c) when mounted on the housing.

14. A cochlear implant headpiece as claimed in any one of claims 1 to 13, further comprising:
a sound processor (103c) within the housing (102c).

15. A cochlear stimulation system, comprising:
a cochlear implant headpiece (100) as claimed in any one of claims 1 to 14 and cochlear implant (10) including a cochlear implant magnet (22) and a cochlear implant antenna (20), or
a cochlear implant headpiece (100) as claimed in any one of claims 1 to 13 and a sound processor (300) including a housing (302) and sound processor circuitry (304) carried within the housing (302) and operably connected to the headpiece antenna (120).

## Patentansprüche

1. Cochleaimplantat-Kopfstück zur Verwendung mit einem Cochleaimplantat, mit:
einem Gehäuse (102, 102c) mit einer oberen Wand (106), einer unteren Wand (104) und einer Aufnahme (116, 116c), welche sich von der oberen Wand zu der unteren Wand erstreckt, ein offenes oberes Ende (140), ein offenes unteres Ende (142) sowie eine zentrale Achse A festlegt und ein Aufnahmeverriegelungsbauteil (142) aufweist;
einer Magnetvorrichtung (114-114‴, 114a, 114a', 114b, 114c, 214, 214a, 214b), welche einen Boden festlegt und einen Magneten (148-148‴, 148a, 148b, 248, 248a, 248b) sowie ein Magnetvorrichtungsverriegelungsbauteil (152) aufweist; und
einer Kopfstückantenne (120) auf dem Gehäuse oder innerhalb des Gehäuses;
wobei die jeweiligen Konfigurationen der Aufnahme (116, 116c) und der Magnetvorrichtung (114-114‴, 114a, 114b, 214, 214a, 214b) so gestaltet sind, dass die Magnetvorrichtung in die Aufnahme eingesetzt werden kann und
die jeweiligen Konfigurationen des Aufnahmeverriegelungsbauteils 142 und des Magnetvorrichtungsverriegelungsbauteils 152 so gestaltet sind, dass die vollständig eingesetzte Magnetvorrichtung in einer Rotationsorientierung um die zentrale Achse aus einer Mehrzahl von Rotationsorientierungen um die zentrale Achse (A) fixiert wird,
**dadurch gekennzeichnet, dass** das Kopfstück so ausgebildet ist, dass, wenn die Magnetvorrichtung vollständig in die Aufnahme eingesetzt ist, der Magnetvorrichtungsboden innerhalb oder nach unten jenseits des offenen unteren Endes der Aufnahme angeordnet ist.

2. Cochleaimplantat-Kopfstück gemäß Anspruch 1, wobei die Magnetvorrichtung (114-114‴, 114a, 114a', 114b, 114c, 214, 214a, 214b) bezüglich der Magnetaufnahme (116, 116c) nicht drehbar ist, wenn sie vollständig in die Aufnahme eingesetzt ist.

3. Cochleaimplantat-Kopfstück gemäß einem der Ansprüche 1 oder 2, wobei, wenn vollständig in die Aufnahme eingesetzt, der Magnetvorrichtungsboden (168b) nach unten jenseits des offenen unteren Endes (142) der Aufnahme angeordnet ist.

4. Cochleaimplantat-Kopfstück gemäß einem der Ansprüche 1-3, wobei die Magnetvorrichtung (114-114‴, 114a, 114a', 114b, 114c, 214, 214a, 214b) ein Gehäuse (150, 150a, 150b) sowie eine Abdeckung (162) aufweist und der Magnet (148a-148‴, 148a, 148b, 248, 248a, 248b) innerhalb des Gehäuses angeordnet ist.

5. Cochleaimplantat-Kopfstück gemäß einem der Ansprüche 1-4, wobei
die Magnetvorrichtung (114-114‴, 114a, 114a', 114b, 114c, 214, 214a, 214b) einen sich in radialer Richtung erstreckenden Flansch (166, 166a, 166b) aufweist;
das Aufnahmeverriegelungsbauteil (146) eine obere Fläche (156) aufweist; und
die jeweiligen Konfigurationen der Aufnahme (116, 116c) und der Magnetvorrichtung so ausgebildet sind, dass der sich in radialer Richtung erstreckende Flansch an der oberen Fläche (156) des Aufnahmeverriegelungsbauteils anliegt, wenn die Magnetvorrichtung vollständig in die Aufnahme eingesetzt ist.

6. Cochleaimplantat-Kopfstück gemäß einem der Ansprüche 1-5, wobei
das Aufnahmeverriegelungsbauteil (146) eine Mehrzahl von Schlitzen (158) aufweist; und
das Magnetvorrichtungsverriegelungsbauteil (152) einen Vorsprung aufweist.

7. Cochleaimplantat-Kopfstück gemäß einem der Ansprüche 1-6, wobei das Gehäuse (102, 102c) einen ringartigen Abschnitt (108) ausweist und eine Schaltung (118, 103c) innerhalb des ringartigen Abschnitts beinhaltet.

8. Cochleaimplantat-Kopfstück gemäß Anspruch 7, wobei die Kopfstückantenne (120, 109c) innerhalb des im Wesentlichen ringartigen Abschnitts (108) angeordnet ist.

9. Cochleaimplantat-Kopfstück gemäß einem der Ansprüche 1-8, wobei
die obere Wand (106) des Gehäuses eine obere Gehäusefläche festlegt;
die Magnetvorrichtung (114-114, 214) eine obere Fläche der Magnetvorrichtung festlegt; und
die jeweiligen Konfigurationen der Aufnahme (116, 116c) und der Magnetvorrichtung so ausgebildet sind, dass die obere Fläche der Magnetvorrichtung bündig mit einem benachbarten Abschnitt der oberen Fläche des Gehäuses ist, wenn die Magnetvorrichtung vollständig in die Aufnahme eingesetzt ist.

10. Cochleaimplantat-Kopfstück gemäß einem der Ansprüche 1-8, wobei
die obere Gehäusewand (106) eine obere Fläche des Gehäuses festlegt;
die Magnetvorrichtung (114a, 114a', 114b, 214a, 214b) eine obere Fläche der Magnetvorrichtung festlegt; und
die jeweiligen Konfigurationen der Aufnahme (116) und der Magnetvorrichtung so gestaltet sind, dass die obere Fläche der Magnetvorrichtung außerhalb jenseits eines benachbarten Abschnitts der oberen Fläche des Gehäuses angeordnet ist, wenn die Magnetvorrichtung vollständig in die Aufnahme eingesetzt ist.

11. Cochleaimplantat-Kopfstück gemäß einem der Ansprüche 1-10, wobei der Kopfstückmagnet (148-148‴, 148a, 148a', 148b) einen diametral magnetisierten Magneten aufweist.

12. Cochleaimplantat-Kopfstück gemäß einem der Ansprüche 1-10, wobei der Kopfstückmagnet (248, 248a, 248b) einen axial magnetisierten Magneten aufweist.

13. Cochleaimplantat-Kopfstück gemäß einem der Ansprüche 1-12, ferner versehen mit einer Kappe (112, 112c), die ausgebildet ist, um auf dem Gehäuse (102, 102c) montiert zu werden und die Magnetaufnahme (116, 116c) abzudecken, wenn sie auf dem Gehäuse montiert ist.

14. Cochleaimplantat-Kopfstück gemäß einem der Ansprüche 1-13, ferner versehen mit einem Schallprozessor (103c) innerhalb des Gehäuses (102c).

15. Cochleastimulationssystem mit:
einem Cochleaimplantat-Kopfstück gemäß einem der Ansprüche 1-14 und einem Cochleaimplantat (10), einschließlich eines Cochleaimplantatmagneten (22) und einer Cochleaimplantatantenne, oder
einem Cochleaimplantat-Kopfstück (100) gemäß einem der Ansprüche 1-13 und einem Schallprozessor (300) einschließlich eines Gehäuses (302) und einer Schallprozessorschaltung (304), die innerhalb des Gehäuses (302) getragen wird und in Wirkverbindung mit der Kopfstückantenne (120) steht.

## Revendications

1. Tête d'implant cochléaire destinée à être utilisée avec un implant cochléaire, la tête d'implant cochléaire comprenant :
un logement (102, 102c) comprenant une paroi supérieure (106), une paroi inférieure (104) et un réceptacle (116, 116c) qui s'étend de la paroi supérieure à la paroi inférieure, qui définit une extrémité supérieure ouverte (140), une extrémité inférieure ouverte (142) et un axe central (A), et qui comprend un élément de verrouillage de réceptacle (146) ;
un appareil magnétique (114-114‴, 114a, 114a', 114b, 114c, 214, 214a, 214b) définissant un fond et comportant un aimant (148-148‴, 148a, 148b, 248, 248a, 248b) et un élément de verrouillage d'appareil magnétique (152) ; et
une antenne de tête (120) sur ou dans le logement ;
dans lequel
les configurations respectives du réceptacle (116, 116c) et de l'appareil magnétique (114-114‴, 114a, 114c, 214, 214a, 214b) sont tels que l'appareil magnétique peut être inséré dans le réceptacle, et
les configurations respectives de l'élément de verrouillage de réceptacle (146) et de l' élément de verrouillage d'appareil magnétique (152) étant telles que l'appareil magnétique complètement inséré sera fixé dans une orientation parmi une pluralité d'orientations de rotation autour de l'axe central (A),
**caractérisé en ce que** ladite tête est configurée de telle sorte que, lorsque l'appareil magnétique est complètement inséré dans le réceptacle, le fond (168, 168a, 168b) de l'appareil magnétique est situé à l'intérieur ou vers le bas au-delà de l'extrémité inférieure ouverte (142) du réceptacle.

2. Tête d'implant cochléaire selon la revendication 1, l'appareil magnétique (114-114‴, 114a, 114a', 114b, 114c, 214, 214a, 214b) ne pouvant pas tourner par rapport au réceptacle d'aimant (116, 116c) lorsqu'il est complètement inséré dans le réceptacle.

3. Tête d'implant cochléaire selon la revendication 1 ou la revendication 2,
lorsqu'il est complètement inséré dans le réceptacle (116), le fond (168b) de l'appareil magnétique étant situé vers le bas au-delà de l'extrémité inférieure ouverte (142) du réceptacle.

4. Tête d'implant cochléaire selon l'une quelconque des revendications 1 à 3,
l'appareil magnétique (114-114‴, 114a, 114a', 114b, 114c, 214, 214a, 214b) comprenant un boîtier (150, 150a, 150b) et un couvercle (162) et l'aimant (148-148‴, 148a, 148b, 248, 248a, 248b) étant situé à l'intérieur du boîtier.

5. Tête d'implant cochléaire selon l'une quelconque des revendications 1 à 4,
l'appareil magnétique (114-114‴, 114a, 114a', 114b, 114c, 214, 214a, 214b) comprenant une bride (166, 166a, 166b) s'étendant radialement ;
l'élément de verrouillage de réceptacle (146) définissant une surface supérieure (156) ; et
les configurations respectives du réceptacle (116, 116c) et de l'appareil magnétique étant telles que la bride s'étendant radialement vient buter contre la surface supérieure (156) de l'élément de verrouillage de réceptacle lorsque l'appareil magnétique est complètement inséré dans le réceptacle.

6. Tête d'implant cochléaire selon l'une quelconque des revendications 1 à 5,
l'élément de verrouillage de réceptacle (146) comprenant une pluralité de fentes (158) ; et
l'élément de verrouillage d'appareil magnétique (152) comprenant une saillie.

7. Tête d'implant cochléaire selon l'une quelconque des revendications 1 à 6,
le logement (102, 102c) définissant une partie annulaire (108) et comprenant un circuit (118, 103c) à l'intérieur de la partie annulaire.

8. Tête d'implant cochléaire selon la revendication 7, l'antenne de tête (120, 109c) étant située à l'intérieur de la partie sensiblement annulaire (108).

9. Tête d'implant cochléaire selon l'une quelconque des revendications 1 à 8,
la paroi supérieure (106) du logement définissant une surface supérieure de logement ;
l'appareil magnétique (114-114‴, 214) définissant une surface supérieure d'appareil magnétique ; et
les configurations respectives du réceptacle (116, 116c) et de l'appareil magnétique étant telles que la surface supérieure de l'appareil magnétique affleure une partie adjacente de la surface supérieure de logement lorsque l'appareil magnétique est complètement inséré dans le réceptacle.

10. Tête d'implant cochléaire selon l'une quelconque des revendications 1 à 8,
la paroi supérieure (106) du logement définissant une surface supérieure de logement ;
l'appareil magnétique (114a, 114a', 114b, 214a, 214b) définissant une surface supérieure d'appareil magnétique ; et
les configurations respectives du réceptacle (116) et de l'appareil magnétique étant telles que la surface supérieure de l'appareil magnétique est située vers l'extérieur au-delà d'une partie adjacente de la surface supérieure de logement lorsque l'appareil magnétique est complètement inséré dans le réceptacle.

11. Tête d'implant cochléaire selon l'une quelconque des revendications 1 à 10,
l'aimant de tête (148-148‴, 148a, 148a', 148b) comprenant un aimant magnétisé diamétralement.

12. Tête d'implant cochléaire selon l'une quelconque des revendications 1 à 10,
l'aimant de tête (248, 248a, 248b) comprenant un aimant magnétisé axialement.

13. Tête d'implant cochléaire selon l'une quelconque des revendications 1 à 12, comprenant en outre :
un capuchon (112, 112c) configuré pour être monté sur le logement (102, 102c) et pour recouvrir le réceptacle d'aimant (116, 116c) lorsqu'il est monté sur le logement.

14. Tête d'implant cochléaire selon l'une quelconque des revendications 1 à 13, comprenant en outre :
un processeur de son (103c) à l'intérieur du logement (102c).

15. Système de stimulation cochléaire, comprenant :
une tête d'implant cochléaire (100) selon l'une quelconque des revendications 1 à 14 et un implant cochléaire (10) comprenant un aimant d'implant cochléaire (22) et une antenne d'implant cochléaire (20), ou
une tête d'implant cochléaire (100) selon l'une quelconque des revendications 1 à 13 et un processeur de son (300) comprenant un logement (302) et un circuit de processeur de son (304) porté à l'intérieur du logement (302) et connecté de manière opérationnelle à l'antenne de tête (120).
